# EUROPÄISCHE PATENTSCHRIFT

(11) **EP 0 778 304 B1**
(45) Veröffentlichungstag und Bekanntmachung des Hinweises auf die Patenterteilung: **18.12.2002**
(21) Anmeldenummer: 96117263.2
(22) Anmeldetag: 28.10.1996
(51) Int. Cl.: C08G 63/08, C08G 63/46, A61K 9/16, A61L 27/00

(54) **Hydrophobe resorbierbare Polyester und deren Verwendung**
Hydrophobic resorbable polyesters and their use
Polyesters hydrophobes et résorbables et leur utilisation

(30) Priorität: 05.12.1995 DE 19545327
(43) Veröffentlichungstag der Anmeldung: 11.06.1997
(73) Patentinhaber: FRAUNHOFER-GESELLSCHAFT ZUR FÖRDERUNG DER ANGEWANDTEN FORSCHUNG E.V., 80636 München (DE)
(72) Erfinder: Rafler, Gerald, Dr. habil., 14473 Potsdam (DE); Jobmann, Monika, Dipl.-Ing., 21614 Buxtehude (DE); Müller, Bernd, Dr. habil., 24220 Flintbek (DE)

(56) Entgegenhaltungen:
- FR-A- 1 561 554
- DATABASE WPI Week 9315 Derwent Publications Ltd., London, GB; AN 93-128818 XP002043932 & JP 05 064 524 A (SHIMADZU CORP) , 19.März 1993

## Beschreibung

Die Erfindung betrifft neue resorbierbare Homo- oder Copolyester hoher Hydrophobie und ihre Verwendung. Diese Polyester können vorzugsweise als Hüll- bzw. Matrixmaterialien in parenteralen Abgabesystemen für spezielle Pharmaka und Impfstoffe mit kontrollierter Wirk- bzw. Impfstoffliberation sowie zur Beschichtung chirurgischer Implantate in der Human- und Veterinärmedizin eingesetzt werden.

Der Einsatz resorbierbarer Polyester in parenteralen Depotarzneiformen mit retardierter bzw. kontrollierter Freisetzung wird vor allem durch ihre Verarbeitbarkeit mittels wirkstoffverträglicher Technologien, ihre Verträglichkeit mit dem eingesetzten Wirkstoff sowie durch ihr Liberations- und Resorptionsverhalten im menschlichen bzw. tierischen Organismus bestimmt. Bei der chirurgischen Applikation stehen die mechanischen Eigenschaften dieser Polyester sowie ihre Änderung durch die Biodegradation im Vordergrund des Interesses. Sowohl die kunststoff- und phannazeutischtechnologischen als auch die applikativen Eigenschaften der als resorbierbares Naht- und Implantatmaterial bzw. als Hüll- oder Matrixmaterial in parenteralen Abgabesystemen eingesetzten Polyester hängen in komplexer Weise von ihrer chemischen Struktur ab. Die bisherigen Bemühungen zur Anpassung der applikativen Eigenschaften dieser Polymeren an die therapeutischen Erfordernisse zielten vor allem auf eine Erhöhung der Hydrophilie bei Erhalt der Biokompatibilität ab. Für, den Bereich der galenischen Applikation dieser Polymeren resultiert diese Zielstellung vor allem aus Forderungen nach einer Beschleunigung des biologischen Abbaus und aus Überlegungen zur besseren Verträglichkeit mit den vorgesehenen, vorwiegend wasserlöslichen Arzneistoffen. Im medizinisch-technischen Bereich galt die bisherige Entwicklung vor allem der Präparation von resorbierbaren Kunststoffen höherer Flexibilität sowie höheren Festigkeiten.

Höhere Anforderungen an die Bioverträglichkeit von Implantaten, vor allem bei Endoprothesen sowie neuere Entwicklungen bei den verfügbaren Wirkstoffen, beispielsweise bei pharmakologisch aktiven Peptiden und Proteinen erfordern die Bereitstellung von neuen resorbierbaren Polymeren mit neuen applikationsorientiert angepaßten Eigenschaftsbildern.

Üblicherweise werden für die Arzneistoffgalenik binäre Copolyester der D.L-Milch- und der Glykolsäure eingesetzt (vgl. beispielsweise G.W. Hastings, P. Dualaque: Macromolecular Materials, CRC Press, 1984 und M. Szycher: High Performance Biomaterials, Technomic Publishing Co. Inc., 1991). Auch andere Kombinationen von 2- bzw. endständigen Hydroxycarbonsäuren werden in der Fachliteratur beschrieben (vgl. R. Mank, G. Rafler, B. Nerlich: Pharmazie 46 (1991) 9).

Zur Modifizierung der Applikationseigenschaften werden neben den Hydroxycarbonsäuren als Copolymerkomponenten vor allem Polyethylenglykole, bifunktionelle hydroxyl-, amino- bzw. carboxylgruppenhaltige Verbindungen sowie Monosaccharide und ihre Reduktionsprodukte (Zuckeralkohole, wie Mannitol oder Sorbitol) angewandt.

Die in der DE-OS 3 430 852 beschriebene Verwendung mehrwertiger Alkohole (Monosaccharide, Zuckeralkohole) zur Modifizierung von Polylactiden und Poly(lactid-co-glycolid)en zielt vor allem auf die Synthese von sternförmigen Polymeren mit indikationsgerechten Liberationsprofilen für peptidische Wirkstoffe bei ausreichend rascher Resorbierbarkeit. Zur Vermeidung sehr niedrigmolekularer bzw. hochvernetzter Produkte sind die Modifizierungskomponenten mit bis zu 0,2 % nur in geringen Konzentrationen im Polymeren enthalten.

In der EP-PS 0108912 werden polyethylenglykolhaltige Polyglykolide mit bis zu 20 Masse-% Modifizierungskomponente beschrieben, die durch Umesterung des Polyesters mit dem Polyethylenoxid (Mₙ = 4.000 und 1.000) in schmelzflüssiger Phase hergestellt werden. Zur Vermeidung von Abbauprozessen bei der Umesterung werden der Schmelze Orthocarbonate zugesetzt.

Triblockcopolymere des Poly-L-Lactids mit höhermolekularem Polyethylenglykol (Mₙ = 35.000) lassen sich durch katalysatorfreie Ringöffnungspolymerisation von L,L-Dilactid in Gegenwart des Polyethers als Initiator darstellen. Der Polyethergehalt der Materialien wird mit 27 - 81 Mol-% angegeben, wobei mit zunehmendem Gehalt an Polyethylenglykol die Molmasse der Blockcopolyester zwangsläufig abnimmt (P. Cerrai, M. Tricoli: Makromolekulare Chem. Rapid Commun. 14 (1993) 529).

Ausgehend hiervon ist es die Aufgabe der vorliegenden Erfindung, molekular hydrophobierte resorbierbare Polyester für die Wirkstoffgalenik und als Biomaterial für temporäre Implantate vorzuschlagen.

Die Aufgabe wird durch die kennzeichnenden Merkmale des Patentanspruches 1 gelöst. Die Verwendung der erfindungsgemäßen Polyester ist durch die kennzeichnenden Merkmale der Patentansprüche 8 und 9 charakterisiert.

Erfindungsgemäß wird hiermit vorgeschlagen, resorbierbare Homo- oder Copolyester mit Fettalkoholen, Fettsäuren oder deren Derivaten umzusetzen. Bevorzugt wird dabei so vorgegangen, daß Fettsäureester der resorbierbaren Homo- oder Copolyester eingesetzt werden, die durch Kettenstart- und/oder Kettenabbruchreaktionen mit langkettigen Fettalkoholen bzw. Fettsäuren bei der Ringöffnungspolymerisation in schmelzflüssiger Phase bzw. durch polymeranaloge Umsetzungen an den Hydroxyl- und/oder Carboxylendgruppen mit langkettigen Fettsäuren bzw. ihren höheraktiven Derivaten und/oder Fettalkoholen bzw. Fettalkoholderivaten in Suspension erhalten werden.

Als langkettige Fettalkohole und Fettsäuren eignen sich erfindungsgemäß alle gesättigten und einfach bzw. mehrfach ungesättigten Fettalkohole und Fettsäuren sowie Hydroxyfettsäuren mit mehr als 16 Kohlenstoffatomen.

Bevorzugte gesättigte Fettalkohole sind Cetyl-, Stearyl-, Ceryl- und Myricylalkohol. Von den ungesättigten Vertretern ist Oleylalkohol besonders geeignet.

Bevorzugte langkettige Fettsäuren sind Stearin-, Cerotin-, Melissinsäure als gesättigte und Öl- sowie Erucasäure als ungesättigte Fettsäuren.

Als ungesättigte höhere Hydroxycarbonsäure ist die Ricinolsäure besonders geeignet, da sie mit Hydroxyl- und Carboxylgruppe sowohl über die Kettenstart- als auch über die Kettenabbruchfunktion verfügt.

Die molekulare Hydrophobierung mit höheren Fettsäuren bzw. Fettalkoholen ist dabei für alle bekannten resorbierbaren Homo- und Copolyester sowie für die Ringöffnungspolymerisation aller bekannten cyclischen Ester und Diester anwendbar. Auf die erfindungsgemäße Weise können insbesondere Poly-L- und Poly-D,L-lactid, Polytrimethylencarbonat sowie Poly(lactid-coglycolid)e unterschiedlichster Comonomerzusammensetzung hydrophobiert werden.

Erfindungsgemäß erfolgt die Darstellung monosubstituierter hydrophober resorbierbarer Homo- und Copolyester via Ringöffnungspolymerisation durch Zusatz des Fettalkohols zu Polymerisationsbeginn bzw. der Fettsäure zur Beendigung der Polymerisation. Disubstituierte Polyesterderivate werden entsprechend durch zeitlich getrennten Zusatz beider Modifizierungskomponenten erhalten.

Die erfindungstypischen Konzentrationen an Hydrophobierungskomponente liegen bei den durch Ringöffnungspolymerisation hergestellten hydrophoben Polyestern für die monosubstituierten Produkte bei 0,5 - 4 Masse-% und für die disubstituierten bei 1 - 8 Masse-%. Günstige Reaktionstemperaturen liegen dabei im Bereich von 150°C bis 225°C.

Erfindungsgemäß wird gemäß Anspruch 10 weiter vorgeschlagen, eine Veresterung der Endgruppen durchzuführen, dabei sind prinzipiell gleichartige endgruppenmodifizierte resorbierbare Polyester durch Veresterung der Endgruppen mit Fettalkoholen bzw. Fettsäuren in heterogener Phase in für das Polymere ungeeigneten, für die Modifizierungskomponente jedoch geeigneten Lösungsmitteln zugänglich. Zur Erhöhung der Reaktionsgeschwindigkeit sowie zur Erzielung möglichst vollständiger Umsätze an den Endgruppen werden vorzugsweise "aktivierte" Derivate der Modifizierungskomponenten, wie Halogenide, Amide, gemischte Anhydride oder Nitrophenylester bei den Fettsäuren sowie Alkoholate der Alkali- und Erdalkalimetalle bei den Fettalkoholen verwendet. Bevorzugt geeignet sind mit Wasser nicht oder nur begrenzt mischbare Lösungsmittel für die Modifizierungskomponenten, wie aromatische und aliphatische Kohlenwasserstoffe, höhersiedende Ether, Ester und Ketone. Besonders geeignet sind n-Heptan, n-Octan und n-Decan.

Die an den Endgruppen durch langkettige Fettalkohol- und/oder Fettsäureester substituierten resorbierbaren Polyester weisen in Lösung im Bereich von 1,0 - 1,5 ppm ein charakteristisches H-NMR-Signal auf, das zur analytischen Bestimmung des Gehalts an Hydrophobierungskomponente im Polymeren herangezogen werden kann.

Die vorstehend beschriebenen disubstituierten Polyester werden bei Reaktionstemperaturen von 150°C bis 225°C erhalten, wenn die Veresterung in schmelzflüssiger Phase direkt im Anschluß an die Ringöffnungspolymerisation mit den genannten Fettsäuren durchgeführt wird. Die Reaktionszeiten betragen bei dieser Verfahrensweise 1 bis 6 Std. Erfolgt die Veresterung der Endgruppen des Polyesters mit "aktivierten" Fettsäure- bzw. Fettalkoholderivaten in Suspension, so werden Reaktionstemperaturen von 15°C bis 100°C bei Reaktionszeiten von 4 bis 12 Std. angewandt. Vorteilhaft ist dabei, daß die heterogenphasige Veresterung bei Raumtemperatur durchgeführt werden kann.

Die Erfindung wird nachfolgend anhand von Beispielen näher erläutert:

### Ausführungsbeispiele

Als direktes Maß für den Hydrophobierungseffekt wird die Änderung der Grenzflächenspannung im System Methylenchlorid/Wasser genutzt.

**Tabelle 1**

| Grenzflächenspannung im System Methylenchlorid/Wasser in Abhängigkeit vom zugesetzten Polylactid (1 %). | |
|---|---|
| Zusatz | Grenzflächenspannung [mN/m²] |
| ohne | 28,3 |
| PLLA | 21,3 |
| PDLA | 21,0 |
| PDLA/PEG | 18,6 |
| | |
| PLLA: | Poly-L-Lactid |
| PDLA: | Poly-D,L-Lactid |
| PDLA/PEG: | Polyethylenoxidmodifiziertes Poly-D,L-lactid |

Durch die molekulare Hydrophobierung lassen sich vor allem die applikationsrelevanten Eigenschaften, wie das Abbau- und Liberationsverhalten oder die Verträglichkeit mit lipophilen Wirkstoffen, selektiv und gezielt verändern. Struktur und Konzentration des hydrophoben Restes stehen damit neben der Struktur des Polyesterrestes und der Molmasse als weitere Komponente zum applikationsgerechten Tailoring von resorbierbaren Polyestern zur Verfügung.

In organischen Lösungsmitteln, wie aromatische Kohlenwasserstoffe, cyclische Ether, Ketone oder Halogenkohlenwasserstoffe, sind hydrophobierte Homo- und Copolyester in Abhängigkeit von der Struktur des makromolekularen Molekülrestes löslich, und können aus diesen Lösungsmitteln in beliebiger Form, beispielsweise zu Filmen oder Mikropartikeln, verarbeitet werden. Im Gegensatz zu den unmodifizierten bekannten Polymeren gleicher chemischer Struktur lassen sich mit den erfindungsgemäßen resorbierbaren Polyestern nach allen bekannten Verfahren wie Sprühtrocknung, Koacervation, Lösungsmittelverdampfung aus Zwei- bzw. Mehrphasensystemen oder ASES-Prozeß (Aerosol Solvent Extraction System) arzneistoffhaltige Mikropartikel herstellen.

### Beispiel 1

Stearylgruppenhaltige Polylactide (Monosubstitution, (mono-St-PLLA)) 2.000 g L,L-Dilactid werden mit 40 g Stearylalkohol intensiv vermischt und in einem Horizontalkneter mit Austragschnecke in Gegenwart von 0,5*10 exp-4 mol/mol Monomereneinheit Zinn-2-octanoat als Initiator bei 170°C in Masse polymerisiert. Nach 5 h wird die viskose Schmelze über ein Transportband ausgetragen und granuliert. Zur Entfernung des unumgesetzten Monomeren wird das Granulat anschließend mit Methanol extrahiert und bis zur Gewichtskonstanz im Vakuum bei 50°C getrocknet.

Von dem Material werden Stearyl- und Monomergehalt, relative Viskosität (rel. LV) einer 0,5 %-igen Lösung in Dimethylformamid sowie die Veränderung der Grenzflächenspannung im System Methylenchlorid/Wasser bestimmt.

**Tabelle 2**

| Charakteristische Daten des mono-St-PLLA | | | |
|---|---|---|---|
| Monomergehalt [%] | Stearylgehalt [%] | rel.LV | Grenzflächenspannung [mN/m²] |
| 0,4 | 1,9 | 1,10 | 26,1 |

### Beispiele 2-5

Analog Beispiel 1 wurden durch Variation des Stearylalkoholgehalts sowie des bzw. der Monomeren weitere stearylmodifizierte Homo- und Copolyester der Milchsäure hergestellt und aufgearbeitet.

**Tabelle 3**

| Charakteristische Daten verschiedener Homo- und Copolyester der Milchsäure | | | | | |
|---|---|---|---|---|---|
| Beispiel | Produkt | Monomer- | Stearylgehalt [%] | rel.LV gehalt [%] | Grenzflächenspannung [mN/m²] |
| 2 | St-PLLA | 0,2 | 1,0 | 1,25 | 25,9 |
| 3 | | 0,3 | 4,0 | 1,08 | 27,9 |
| 4 | St-PDLA | 0,5 | 2,0 | 1,15 | 26,8 |
| 5 | St-PGDLA | 0,2 | 2,0 | 1,11 | 24,3 |
| St-PGDLA: Poly(glycolid-co-lactid)-stearat St-PLLA: Poly(L-lactid)-stearat ST-PDLA: Poly(D,L-lactid)-stearat | | | | | |

### Beispiel 6

### di-St-PLLA

### Stearylgruppenhaltige Polylactide (Disubstitution)

2.000 g L,L-Dilactid werden mit 40 g Stearylalkohol in einem Horizontalkneter polymerisiert. Nach 5 h werden 45 g Stearinsäure zugesetzt und die Veresterung noch 1 h weitergeführt. Das Polymerisat wird analog Beispiel 1 aufgearbeitet. Die analytische Charakterisierung ergab einen Stearylgruppengehalt von 4,1 %, eine rel. Lösungsviskosität von 1,15 und eine Grenzflächenspannung von 27,8 mN/m.

### Beispiel 7

### mono-Ol-PDLA

### Oleylgruppenhaltige Polylactide (Monosubstitution)

2.000 g D,L-Dilactid werden mit 40 g Oleylalkohol in einem Horizontalkneter bei 130 °C intensiv vermischt. Dann wird das Gemisch auf 150 °C erhitzt und 0.5*10 exp-4 mol/mol Monomereinheit Zinn-2-octonoat zugegeben. Nach einer Polymerisationszeit von 5 h wird das Polymerisat ausgetragen, mit Methanol extrahiert und bei Raumtemperatur im Vakuum konstant getrocknet. NMR-spektroskopisch wurde der Oleylgehalt zu 2,0 % bestimmt. Die rel. Lösungsviskosität einer 0,5 %igen Lösung in Dimethylformamid betrug 1,15.

### Beispiel 8

### Abbauverhalten unmodifizierter und hydrophobierter Polylactide

Zur Charakterisierung des Abbauverhaltens werden jeweils 200 mg wirkstoffhaltiger bzw. wirkstofffreier Mikropartikel, hergestellt mittels eines der genannten Mikropartikelpräparationsverfahren (Sprühtrocknung, Lösungsmittelverdampfung, Koacervation, ASES) bzw. Kryomahlung, in 50 ml wäßrigem Phosphatpuffer bei pH = 7,4 bei 37°C abgebaut. Von den abgebauten, im Vakuum konstant getrockneten Proben werden Masseverlust und relative. Lösungsviskosität als Maß für die Abnahme der Molmasse bestimmt.

In den Tabellen 4-7 sind Ergebnisse solcher Abbauuntersuchungen an wirkstofffreien Partikeln für verschiedene Polylactide zusammengefaßt.

**Tabelle 4**

| In vitro Abbau von PLLA | | |
|---|---|---|
| Zeit [d] | Masseverlust [%] | rel.LV |
| 0 | 0 | 1,16 |
| 14 | 4,5 | 1,16 |
| 28 | 6,4 | 1,14 |
| 42 | 8,0 | 1,14 |
| 90 | 12,9 | 1,13 |

**Tabelle 5**

| In vitro Abbau von mono-St-PLLA | | |
|---|---|---|
| Zeit [d] | Masseverlust [%] | rel.LV |
| 0 | 0 | 1,10 |
| 14 | 0,2 | 1,09 |
| 28 | 0,3 | 1,09 |
| 42 | 0,3 | 1,09 |
| 90 | 2,2 | 1,09 |

**Tabelle 6**

| In vitro Abbau von mono-0L1-PLLA | | |
|---|---|---|
| Zeit [d] | Masseverlust [%] | rel.LV |
| 0 | 0 | 1,13 |
| 14 | 2,3 | 1,12 |
| 28 | 2,6 | 1,12 |
| 42 | 2,6 | 1,12 |
| 90 | 2,7 | 1,12 |

**Tabelle 7**

| In vitro Abbau von di-St-PLLA | | |
|---|---|---|
| Zeit [d] | Masseverlust [%] | rel.LV |
| 0 | 0 | 1,69 |
| 14 | 1,8 | 1,68 |
| 28 | 2,4 | 1,68 |
| 42 | 4,8 | 1,68 |
| 90 | 5,7 | 1,64 |

## Patentansprüche

1. Verfahren zur Herstellung von hydrophobierten resorbierbaren Polyestern für medizinische Anwendungen, bei dem eine Ringöffnungspolymerisation in schmelzflüssiger Phase von cyclischen Estern in Gegenwart eines. Initiators und einer Hydrophobierungskomponente durchgeführt wird,
**dadurch gekennzeichnet,**
**daß** als Hydrophobierungskomponente ein Fettalkohol mit mehr als 16 C-Atomen zur Veresterung eingesetzt wird.

2. Verfahren nach Anspruch. 1,
**dadurch gekennzeichnet, daß** als Fettalkohol Cetyl-, Stearyl-, Ceryl-, Myricyl- oder Oleylalkohol verwendet wird.

3. Verfahren nach Anspruch 1 oder 2
**dadurch gekennzeichnet, daß** nach Abschluß der Ringöffnungspolymerisation eine Veresterung der Endgruppen in schmelzflüssiger Phase oder in Suspension mit einer Fettsäure mit mehr als 16 C-Atomen durchgeführt wird.

4. Verfahren nach Anspruch 3,
**dadurch gekennzeichnet, daß** als Fettsäuren Stearin-, Cerotin-, Melissin-, Öl-, Eruca- oder Ricinoläure eingesetzt wird.

5. Verfahren nach mindestens einem der Ansprüche 1 bis 4,
**dadurch gekennzeichnet, daß** 0,5 bis 10 Masse-% Hydrophobierungskomponente und zu 100 Masse-% ergänzte Anteile an cyclischem Ester eingesetzt werden.

6. Verfahren nach einem der Ansprüche 1 bis 5,
**dadurch gekennzeichnet, daß** die Ringöffnungspolymerisation bei 150 °C bis 225 °C über eine Zeitspanne von 1 Std. bis 6 Std. durchgeführt wird.

7. Verfahren nach einem der Ansprüche 1 bis 6,
**dadurch gekennzeichnet, daß** als cyclische Ester L,L- oder D,L-Dilactid, Diglycolid, Trimethylencarbonat, E-Caprolacton oder deren Mischungen eingesetzt werden.

8. Verwendung der Polyester, hergestellt nach mindestens einem der Ansprüche 1 bis 7, in der Wirkstoffgalenik.

9. Verwendung der Polyester, hergestellt nach mindestens einem der Ansprüche 1 bis 7, zur Beschichtung chirurgischer Implantate.

## Claims

1. Method for producing hydrophobic resorbable polyesters for medicinal applications, in which a ring-opening polymerisation is implemented in a molten phase of cyclic esters in the presence of an initiator and of a hydrophobing component,
***characterised in that***
a fatty alcohol with more than 16 C-atoms is used as hydrophobing component for esterification.

2. Method according to claim 1,
**characterised in that** cetyl-, stearyl-, ceryl-, myricyl- or oleyl alcohol is used as fatty alcohol.

3. Method according to claim 1 or 2,
**characterised in that** esterification of the end groups is implemented in molten phase or in suspension with a fatty acid with more than 16 C-atoms after termination of the ring-opening polymerisation.

4. Method according to claim 3,
**characterised in that** stearic-, cerotin-, melissic-, oleic-, erucic- or ricinoleic acid is used as fatty acids.

5. Method according to at least one of the claims 1 to 4,
**characterised in that** 0.5 to 10% by mass hydrophobing component and up to 100% by mass of supplementary components of cyclic ester are used.

6. Method according to one of the claims 1 to 5,
**characterised in that** the ring-opening polymerisation is implemented at 150°C to 225°C over a timespan of 1 hour to 6 hours.

7. Method according to one of the claims 1 to 6,
**characterised in that** L,L- or D,L-dilactide, diglycolide, trimethylene carbonate, E-caprolactone or mixtures thereof are used as cyclic esters.

8. Use of the polyesters, produced according to at least one of the claims 1 to 7, in active substance galenics.

9. Use of the polyesters, produced according to at least one of the claims 1 to 7, for coating surgical implants.

## Revendications

1. Procédé pour la préparation de polyesters résorbables rendus hydrophobes, pour applications médicales, dans lequel on effectue une polymérisation avec ouverture de cycle en phase liquide d'esters cycliques, en présence d'un amorceur et d'un composant d'hydrofugation,
**caractérisé en ce qu'**
on utilise pour l'estêrification, comme composant d'hydrofugation, un alcool gras ayant plus de 16 atomes de carbone.

2. Procédé selon la revendication 1,
**caractérisé en ce qu'**
on utilise comme alcool gras l'alcool cétylique, stéarylique, cérylique, myricylique ou oléylique.

3. Procédé selon la revendication 1 ou 2,
**caractérisé en ce que**,
une fois terminée la polymérisation avec ouverture de cycle, on effectue une estérification des groupes terminaux en phase liquide ou en suspension, avec un acide gras ayant plus de 16 atomes de carbone.

4. Procédé selon la revendication 3,
**caractérisé en ce qu'**
on utilise comme acides gras l'acide stéarique, cérotique, mélessique, oléique, érucique ou ricinoléique.

5. Procédé selon au moins l'une quelconque des revendications 1 à 4,
**caractérisé en ce qu'**
on utilise de 0,5 à 10 % en masse de composant d'hydrofugation et des parties complétées jusqu'à 100 % en masse d'ester cyclique.

6. Procédé selon l'une quelconque des revendications 1 à 5,
**caractérisé en ce qu'**
on effectue la polymérisation avec ouverture de cycle à 150-225°C, pendant une durée de 1 à 6 heures.

7. Procédé selon l'une quelconque des revendications 1 à 6,
**caractérisé en ce qu'**
on utilise comme éther cyclique le N,N- ou D,L-dilactide, le diglycolide, le carbonate de triméthylène, l'ε-caprolactone ou des mélanges de ceux-ci.

8. Utilisation des polyesters préparés selon au moins l'une quelconque des revendications 1 à 7, dans la préparation de compositions galéniques.

9. Utilisation des polyesters préparés selon au moins l'une quelconque des revendications 1 à 7, pour l'enduction d'implants chirurgicaux.
